# FASCICULE DE BREVET EUROPEEN

(11) **EP 1 980 280 B1**
(45) Date de publication et mention de la délivrance du brevet: **07.11.2018**
(21) Numéro de dépôt: 07290447.7
(22) Date de dépôt: 11.04.2007
(51) Int. Cl.: A61M 1/00, A61M 27/00

(54) **Drain chirurgical**
Chirurgischer Drain
Surgical drain

(43) Date de publication de la demande: 15.10.2008
(73) Titulaire: Lesca, Christophe, 75016 Paris (FR)
(72) Inventeur: Lesca, Christophe, 75016 Paris (FR)
(74) Mandataire: Littolff, Denis

(56) Documents cités:
- DE-U1- 8 815 869
- US-A- 4 579 555
- US-A- 5 599 304

## Description

La présente invention concerne un drain chirurgical aspiratif destiné à être positionné au contact de surfaces que l'on veut drainer à la suite d'un acte chirurgical. De manière générale, un tel acte comporte un risque d'hématome post-opératoire, c'est-à-dire une collection de sang dans un espace de décollement de tissus résultant de l'intervention chirurgicale. Le risque d'hématome est d'autant plus important que la surface de décollement des tissus est importante.

Or, en chirurgie plastique et esthétique, les décollements sont souvent importants, dans la plupart des cas en sous-cutanée et parfois à des niveaux plus profonds dans l'hypothèse d'interventions nécessitant des reconstructions plus complexes.

Actuellement, les techniques de drainage font appel soit à des systèmes passifs, permettant ou favorisant un écoulement passif des fluides à évacuer, soit des systèmes où le drainage se fait par aspiration, faisant appel à un composant de pompage.

Les systèmes passifs sont par exemple des lames de caoutchouc positionnés au contact de la surface à drainer, et dépassant de la zone opérée par l'incision ayant servi à l'intervention, ou par une contre-incision effectuée spécifiquement pour le drainage. Elles doivent être positionnées avec une certaine déclivité par rapport à la région à drainer pour que le liquide biologique puisse s'écouler naturellement. La ou les lames de caoutchouc utilisées sont amarrées aux berges cutanées de l'incision par un fil de suture. Les lames de caoutchouc sont en pratique surtout utilisées dans des hypothèses où une efficacité de drainage maximale est recherchée, par exemple pour évacuer des collections résultant d'abcès. Elles présentent cependant l'inconvénient

d'être relativement larges, et peuvent laisser des cicatrices disgracieuses après leur retrait.

Parmi les systèmes passifs, il faut également mentionner les gros fils de suture appelés crins de Florence, que l'on positionne de part et d'autre d'une incision située au-dessus d'une zone de décollement. Le drainage du sang se fait par capillarité, empêchant la cicatrisation au niveau du point de sortie.

Il existe également des systèmes actifs procédant à un drainage aspiratif, comme par exemple le drain de Redon. Il se compose d'un tuyau percé de trous circulaires dans sa partie active, c'est-à-dire celle qui est positionnée dans la surface à drainer. Ce tuyau comporte une extrémité sans trous que l'on fait passer hors de la zone à drainer à l'aide d'une sorte d'hameçon appelé alêne de Redon, qui permet de lui faire traverser la peau. La partie extracorporelle de ce drain est alors amarrée à la peau par un fil, et est raccordée à une bouteille, en général en matière plastique, dans laquelle un vide a été établi. La pression négative qui règne dans la bouteille permet d'aspirer le sang susceptible de se répandre dans la zone opérée.

Lorsque la surface de décollement à drainer est importante, les chirurgiens doivent positionner autant de drains que de régions que l'on veut drainer, car le drain comporte un tuyau unique. Il est donc nécessaire de disposer de plusieurs flacons ou bouteilles pour recueillir le liquide qui est drainé hors de la surface de décollement. L'implantation des drains / bouteilles peut être fastidieuse, et entraîner une perte de temps car il faut positionner chaque drain l'un après l'autre, puis établir la liaison avec la ou les bouteilles correspondantes.

Par ailleurs, la nécessité d'utiliser plusieurs drains lorsque la surface de décollement est importante a des répercussions économiques évidemment défavorables, compte tenu du prix de revient d'un drain de Redon.

Le document US 5 599 304 propose un drain pour aspirer les voies nasosinusiennes d'un sujet, dans lequel au moins un tube d'aspiration passe à travers la cavité buccale dans les voies nasales, ledit drain pouvant comprendre au moins un noeud de division en au moins deux branches. Toutefois, ce type de drain n'est pas adapté pour être positionné au contact de surfaces que l'on veut drainer à la suite d'un acte chirurgical, notamment pour éviter la survenue d'hématomes post opératoires.

Le document DE8815869U propose un drain pour le drainage abdominal, ledit drain se composant d'un tube de drainage principal qui se ramifie du côté patient en plusieurs tuyaux de drainage secondaires.

La présente invention remédie à ces inconvénients, en proposant un drain original susceptible de drainer plusieurs régions chirurgicales différentes. A cet effet, selon une première variante, le drain chirurgical aspiratif de l'invention comprend :
- un tronc formant une première portion de section maximale;
- au moins un noeud de division du tronc en au moins deux branches, l'angle de raccordement des branches avec le tronc étant aigu;
- le matériau de réalisation du drain étant flexible;
- la division du tronc en n branches étant réalisée par n cloisons d'allure radiale se dédoublant pour séparer les branches;
- le tronc étant cylindrique à section circulaire, la division dudit tronc étant réalisée par des cloisons radiales se rejoignant au niveau de l'axe du cylindre;
- la somme des surfaces des sections des branches étant au plus égale à la surface de la section du tronc, lesdites branches étant inscrites dans le périmètre du cercle constituant la section du tronc.

Selon une seconde variante, le drain chirurgical aspiratif de l'invention comprend:
- un tronc formant une première portion de section maximale;
- au moins un noeud de division du tronc en au moins deux branches, l'angle de raccordement des branches avec le tronc étant aigu;
- le matériau de réalisation du drain étant flexible;
- la section du tronc étant ellipsoïdale, la division du tronc en branches s'effectuant par cloisonnement d'allure transversale;
- la somme des surfaces des sections des branches étant au plus égale à la surface de la section du tronc, lesdites branches étant inscrites dans le périmètre de l'ellipsoïde constituant la section du tronc.

La configuration du drain reflète donc une arborescence offrant une surface de drainage largement accrue et permettant que s'établisse une pression aspirative plus harmonieuse, améliorant par conséquent les performances des drains existants, surtout dans les actes chirurgicaux donnant lieu à d'importants décollements tissulaires. C'est par exemple le cas en chirurgie plastique et reconstructrice, domaines dans lesquels les opérations peuvent présenter des décollements sous-cutanés importants.

En fait, schématiquement, le drain de l'invention est constitué d'un axe central sur lequel se greffe à angle aigu des axes secondaires à la manière des branches d'un arbre. Dans une première version, la division du tronc en n branches est réalisé par n cloisons d'allure radiale se dédoublant pour séparer les branches.

Dans cette hypothèse, le tronc est cylindrique à section circulaire, la division dudit tronc étant réalisée par des cloisons radiales se rejoignant au niveau de l'axe du cylindre.

Selon une seconde variante, la section du tronc peut être ellipsoïdale, la division en branches s'effectuant par cloisonnement d'allure transversale. Dans ce cas, selon une possibilité, chaque branche peut initialement présenter une section reconstituant une ellipse de grand axe d'allure parallèle au petit axe de l'ellipse originelle du tronc.

Afin de ne pas augmenter le diamètre du drain du fait de la présence des branches latérales, et afin de permettre un retrait du drain dans de bonnes conditions, il est impératif que l'angle de raccordement des axes ou branches secondaires avec l'axe ou tronc central soit aigu. Par ailleurs, selon une configuration possible, les branches contiguës peuvent présenter l'une une paroi convexe et l'autre une paroi concave prévues pour s'imbriquer sur au moins une partie de leur longueur.

Ces parois peuvent être de forme ellipsoïdale. Dans cette hypothèse, de préférence, l'enveloppe extérieure de la branche dans laquelle une division est réalisée est elle-même de section ellipsoïdale au niveau de ladite division.

Par ailleurs, il est à noter que les branches issues d'une séparation ont une section qui devient progressivement circulaire.

Le drain de l'invention doit bien entendu être fabriqué en matière plastique souple, par exemple en silicone.

Dans la plupart des cas, le diamètre des branches est inférieur au tuyau unique du drain de Redon. Cela étant, la rançon cicatricielle étant peu importante au niveau de la sortie cutanée du drain, il est parfaitement possible de prendre un diamètre initial se rapprochant de celui du drain du tuyau de Redon. Par ailleurs, la fabrication en silicone autorise un diamètre légèrement inférieur pour des performances égales, car le silicone diminue l'agrégabilité plaquettaire au niveau des trous de drainage. Par conséquent, les risques de formation de caillots sanguins obstruant le drain sont largement diminués.

Dans le cas précité où deux branches peuvent s'emboîter parce que l'une présente un relief en creux, celle qui présente une paroi convexe peut être plus longue que celle qui présente le relief en creux concave. Il suffit, pour ne pas créer de problèmes au retrait dus à une augmentation brutale de section, de retirer d'abord la branche la plus longue dotée de la paroi ellipsoïdale convexe de quelques centimètres, avant de retirer le restant du drain.

Il est à noter que le drain, destiné à être vissé à une alêne de Redon, doit donc présenter à son extrémité libre une section circulaire permettant son vissage. Pour les drains qui comportent un tronc à section ellipsoïdale, celle-ci est donc toujours précédée d'une telle section circulaire. La surface de ces sections doit être identique pour que leur passage transcutané se fasse dans de bonne conditions.

Bien entendu, l'invention autorise des configurations multiples, à deux, trois (voire plus) noeuds selon les utilisations, et en particulier selon la surface de décollement à drainer.

L'invention va à présent être décrite plus en détail, en référence aux figures annexées, pour lesquelles :
- la figure 1 représente un drain selon l'invention à un noeud et trois branches ;
- les figures 2a à 2e montrent différents plans de coupe correspondant aux flèches a à e dans la figure 1 ;
- la figure 3 représente un drain à deux noeuds et trois branches selon la seconde variante présentée auparavant ;
- les figures 4a à 4g représentent des plans de coupe du drain de la figure 3 selon les flèches a à g ;
- la figure 5 illustre un exemple de drain selon la seconde variante à un noeud et deux branches ; et
- les figures 6a à 6f montrent des plans de coupe du drain de la figure 5 selon les flèches a à f.

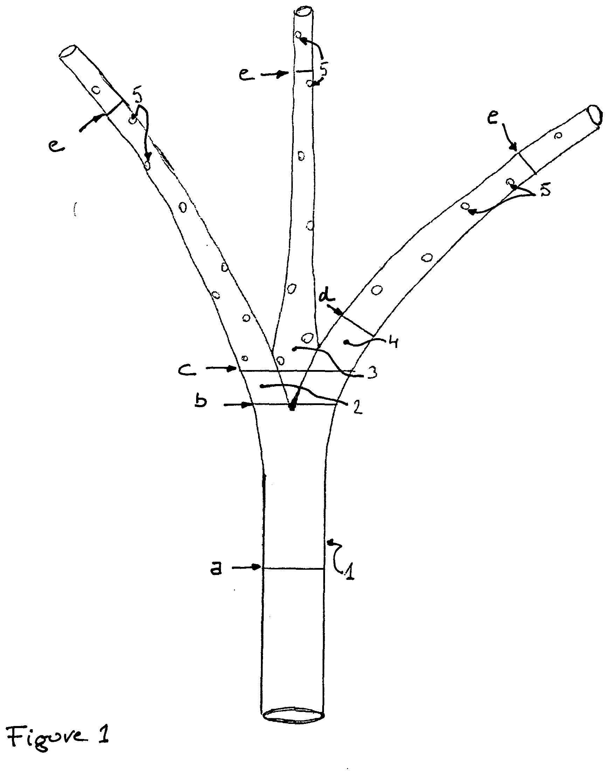

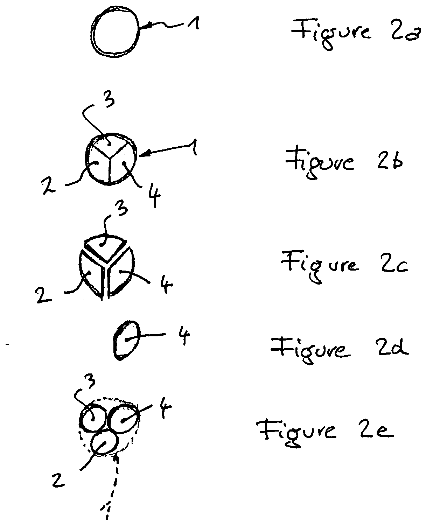

Le drain de la figure 1, que l'on peut qualifier de "drain palmier" comporte un tronc central (1) et trois branches (2, 3, 4) dotées chacune d'orifices (5) de drainage. Le processus de division de l'arbre (1) en des branches (2, 3, 4) est montré au figures 2a à 2e. La figure 2a montre la section du tronc principal (1), prise au niveau de la flèche a, alors que la figure 2b montre l'amorce du cloisonnement radial prévue pour aboutir à la division en trois branches (2) à (4). Au niveau de la flèche b, le cloisonnement est effectué, mais les branches (2, 3, 4) non encore séparées.

Cette division apparaît en figure 2c, les cloisons radiales se dédoublant (au niveau de la flèche c) pour permettre la séparation des branches (2, 3, 4). Progressivement, la section de chacune des branches s'arrondit, comme cela est montré en figure 2d, qui montre la section au niveau de la flèche d de la figure 1.

La section prise au niveau de la flèche e de la figure 1 est complètement circulaire, comme le montre la figure 2e qui illustre au surplus la condition selon laquelle les trois branches de section circulaire doivent être inscrites dans le périmètre du cercle constituant la section du tronc principal (1).

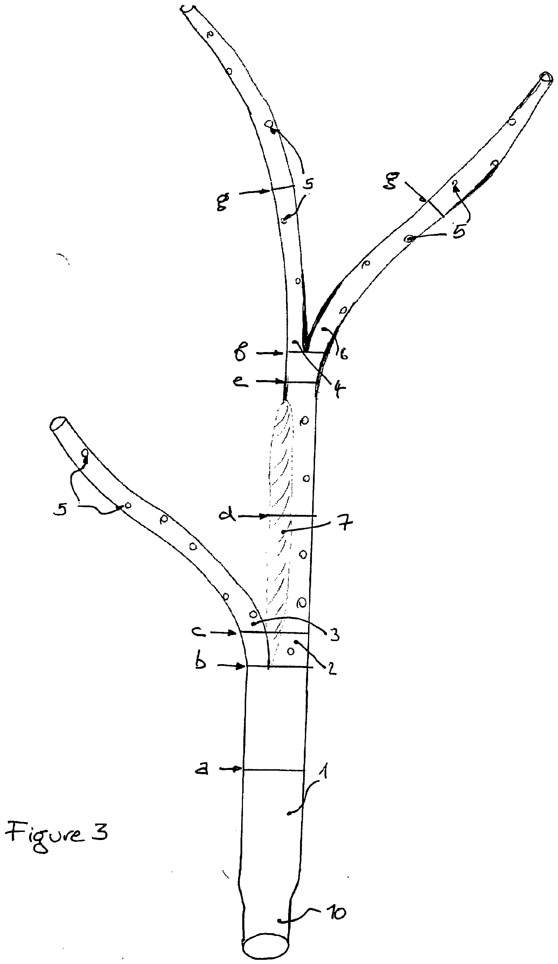

La figure 3 montre un exemple de la seconde variante de l'invention, mettant en oeuvre des drains à plusieurs noeuds. Dans l'hypothèse représentée, il s'agit d'un drain à deux noeuds et trois branches.

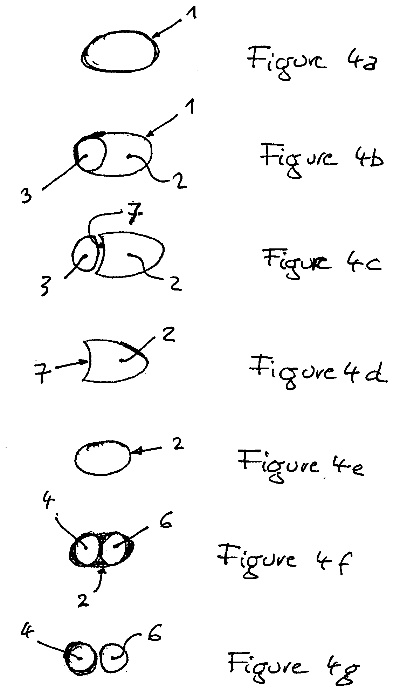

Cette figure doit également être décrite à la lumière des figures 4a à 4g qui permettent de comprendre la division successive en plusieurs branches. Dans cette hypothèse, le plan de coupe du tronc central (1), avant le premier noeud, est ellipsoïdal, et fait suite à une portion de section circulaire (10) pour permettre le vissage à une alêne de Redon.

Au niveau de la section montrée par la flèche b, et montrée en figure 4b, une première séparation s'amorce en une branche principale (2) et une branche latérale (3), cette dernière présentant également une section ellipsoïdale, mais de grand axe parallèle au petit axe de l'ellipsoïde du tronc principal (1).

La séparation est effectuée au niveau de la flèche c, dont la représentation apparaît en figure 4c. A ce stade, les branches (2) et (3) sont séparées, et la branche (2) comporte une surface latérale concave (7) qui épouse une surface extérieure convexe correspondante de la branche (3).

La branche (2) seule est montrée en figure 4d, qui correspond à la section de la flèche d en figure 3. En principe, la surface concave (7) n'existe que tant qu'il y a une branche latérale (3) susceptible d'être plaquée contre elle, de manière à recréer le volume initial du tronc (1). Cela veut dire que, au-delà de la longueur effective de la branche latérale (2), la section de la branche principale (2) en portion d'ellipse avec une surface concave (7) n'a plus de raison d'être. C'est ce qui apparaît en figure 4e, illustrant la section représentée par la flèche e en figure 3.

Celle-ci peut à nouveau se diviser en deux, au niveau du noeud figuré par la flèche f, la section correspondante étant montrée en figure 4f. Il y a alors deux nouvelles branches (4) et (6) qui sont issues de la branche principale (2). Les deux nouvelles branches sont également ellipsoïdales, de grand axe correspondant sensiblement au petit axe de la branche principale (2).

Quelques centimètres après la séparation, la section de chacune des branches (4) et (6) devient sensiblement circulaire, au niveau de la flèche g, comme cela apparaît en figure 4g.

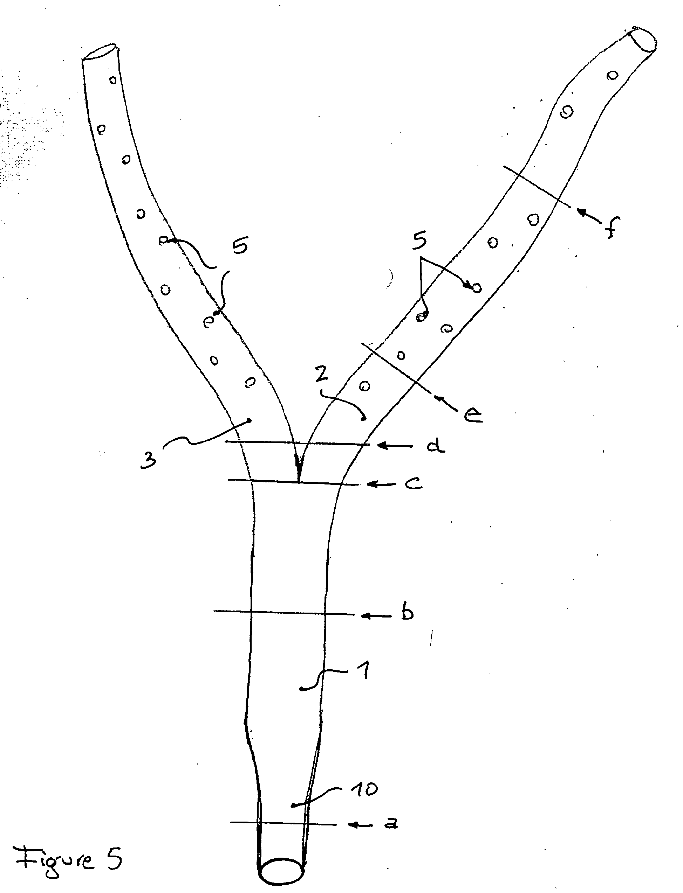

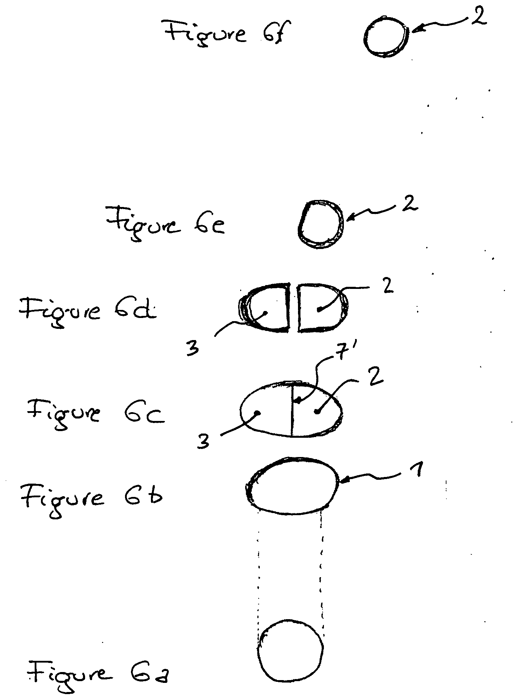

Une autre variante encore apparaît en figures 5 et 6a à 6f. Dans ce cas, le schéma de division suivi obéit à la même logique qu'auparavant, mais avec une division par cloison transversale (7') d'allure rectiligne (voir en figure 6c correspondant à la flèche c de la figure 5). A mesure que les branches s'éloignent, elles prennent une section circulaire.

Les explications ci-dessus ne concernent que des exemples possibles de l'invention, qui n'en sont pas exhaustifs. Au contraire, l'invention englobe les variantes de forme comprenant par exemple l'existence de noeuds supplémentaires dans les branches secondaires de la figure 1, ou l'adjonction d'un troisième noeud dans la variante de la figure 3, etc.

## Revendications

1. Drain chirurgical aspiratif comprenant:
- un tronc (1) formant une première portion de section maximale;
- au moins un noeud de division du tronc (1) en au moins deux branches (2, 3, 4), l'angle de raccordement des branches (2, 3, 4) avec le tronc (1) étant aigu;
- le matériau de réalisation du drain étant flexible;
- la division du tronc (1) en n branches (2, 3, 4) étant réalisée par n cloisons d'allure radiale se dédoublant pour séparer les branches (2, 3, 4);
- le tronc (1) étant cylindrique à section circulaire, la division dudit tronc (1) étant réalisée par des cloisons radiales se rejoignant au niveau de l'axe du cylindre;
**caractérisé en ce que** la somme des surfaces des sections des branches (2, 3, 4) est au plus égale à la surface de la section du tronc (1), lesdites branches (2, 3, 4) étant inscrites dans le périmètre du cercle constituant la section du tronc (1).

2. Drain chirurgical aspiratif comprenant:
- un tronc (1) formant une première portion de section maximale;
- au moins un noeud de division du tronc (1) en au moins deux branches (2, 3, 4), l'angle de raccordement des branches (2, 3, 4) avec le tronc (1) étant aigu;
- le matériau de réalisation du drain étant flexible;
- la section du tronc (1) étant ellipsoïdale, la division du tronc (1) en branches (2, 3, 4) s'effectuant par cloisonnement d'allure transversale;
**caractérisé en ce que** la somme des surfaces des sections des branches (2, 3, 4) est au plus égale à la surface de la section du tronc (1), lesdites branches (2, 3, 4) étant inscrites dans le périmètre de l'ellipsoïde constituant la section du tronc (1).

3. Drain chirurgical aspiratif selon la revendication précédente, **caractérisé en ce que** les branches (2, 3, 4) issues de la division ont une section reconstituant une ellipse de grand axe d'allure parallèle au petit axe de l'ellipse du tronc (1).

4. Drain chirurgical aspiratif selon la revendication précédente, **caractérisé en ce que** deux branches (2, 3) contiguës présentent l'une une paroi convexe et l'autre une paroi concave (7) prévues pour s'imbriquer sur au moins une partie de leur longueur.

5. Drain chirurgical aspiratif selon l'une quelconque de revendications précédentes, **caractérisé en ce que** les branches (2, 3, 4) issues d'une séparation ont une section qui devient progressivement circulaire.

6. Drain chirurgical aspiratif selon l'une quelconque des revendications précédentes, **caractérisé en ce qu'**il est fabriqué en matière plastique souple, de type silicone.

7. Drain chirurgical aspiratif selon l'une quelconque de revendications précédentes, **caractérisé en ce que** le tronc (1) comporte une portion d'extrémité (10) à section circulaire.

## Patentansprüche

1. Chirurgischer Ansauge-Drain, umfassend :
- einen Schaft (1), der einen ersten Abschnitt mit maximalem Querschnitt bildet;
- mindestens einen Knoten zum Teilen des Schafts (1) in mindestens zwei Zweige (2, 3, 4), wobei der Kontaktwinkel der Zweige (2, 3, 4) mit dem Schaft (1) spitz ist;
- wobei das Material, aus dem der Drain gefertigt ist, biegsam ist;
- wobei die Teilung des Schafts (1) in n Zweige (2, 3, 4) durch n radiale Trennwände erfolgt, die sich aufteilen, um die Zweige (2, 3, 4) zu trennen;
- wobei der Schaft (1) zylindrisch ist und einen kreisförmigen Querschnitt aufweist, wobei die Teilung des Schafts (1) durch radiale Trennwände erfolgt, die auf der Höhe der Achse des Zylinders zusammenlaufen;
**dadurch gekennzeichnet, dass** die Summe der Querschnittsfläche der Zweige (2, 3, 4) höchstens gleich der Querschnittsfläche des Schafts (1) ist, wobei die Zweige (2, 3, 4) im Umfang des Kreises angeordnet ist, der vom Querschnitt des Schafts (1) ausgebildet wird.

2. Chirurgischer Ansauge-Drain, umfassend :
- einen Schaft (1), der einen ersten Abschnitt mit maximalem Querschnitt bildet;
- mindestens einen Knoten zum Teilen des Schafts (1) in mindestens zwei Zweige (2, 3, 4), wobei der Kontaktwinkel der Zweige (2, 3, 4) mit dem Schaft (1) spitz ist;
- wobei das Material, aus dem der Drain gefertigt ist, biegsam ist;
- wobei der Querschnitt (1) des Schafts ellipsoid ist, und wobei die Teilung des Schafts (1) in Zweige (2, 3, 4) durch querlaufende Trennwände erfolgt;
**dadurch gekennzeichnet, dass** die Summe der Querschnittsfläche der Zweige (2, 3, 4) höchstens gleich der Querschnittsfläche des Schafts (1) ist, wobei die Zweige (2, 3, 4) im Umfang des Ellipsoids angeordnet ist, der vom Querschnitt des Schafts (1) ausgebildet wird.

3. Chirurgischer Ansauge-Drain nach dem vorstehenden Anspruch, **dadurch gekennzeichnet, dass** die aus der Teilung entstehenden Zweige (2, 3, 4) einen Querschnitt aufweisen, der eine Ellipse bildet, deren Hauptachse parallel zur Nebenachse der Ellipse des Schafts (1) verläuft.

4. Chirurgischer Ansauge-Drain nach dem vorstehenden Anspruch, **dadurch gekennzeichnet, dass** zwei angrenzende Zweige (2, 3) jeweils eine konvexe Wand bzw. konkave Wand (7) aufweisen, die zum Ineinandergreifen über mindestens einen Teil ihrer Länge vorgesehen sind.

5. Chirurgischer Ansauge-Drain nach einem der vorstehenden Ansprüche, **dadurch gekennzeichnet, dass** die aus einer Teilung entstehenden Zweige (2, 3, 4) einen Querschnitt aufweisen, der allmählich kreisförmig wird.

6. Chirurgischer Ansauge-Drain nach einem der vorstehenden Ansprüche, **dadurch gekennzeichnet, dass** er aus einem weichen Kunststoff in der Art von Silikon besteht.

7. Chirurgischer Ansauge-Drain nach einem der vorstehenden Ansprüche, **dadurch gekennzeichnet, dass** der Schaft (1) einen Endabschnitt (10) mit kreisförmigem Querschnitt umfasst.

## Claims

1. Surgical suction drain comprising:
- a stem (1) forming a first portion of maximum section;
- at least one node for dividing the stem (1) into at least two branches (2, 3, 4), the connecting angle of the branches (2, 3, 4) with the stem (1) being acute;
- the material for producing the drain being flexible;
- the division of the stem (1) into n branches (2, 3, 4) being done by n radial partitions splitting to separate the branches (2, 3, 4);
- the stem (1) being cylindrical with a circular section, the division of said stem (1) being done by radial partitions coming together at the axis of the cylinder;
**characterized in that** the sum of the surfaces of the sections of the branches (2, 3, 4) is at most equal to the surface of the section of the stem (1), said branches (2, 3, 4) being inscribed in the perimeter of the circle making up the section of the stem (1).

2. Surgical suction drain, comprising:
- a stem (1) forming a first portion of maximum section;
- at least one node for dividing the stem (1) into at least two branches (2, 3, 4), the connecting angle of the branches (2, 3, 4) with the stem (1) being acute;
- the material for producing the drain being flexible;
- the section of the stem (1) being elliptical, the division of the stem (1) into branches (2, 3, 4) being done by transverse partitioning;
**characterized in that** the sum of the surfaces of the sections of the branches (2, 3, 4) is at most equal to the surface of the section of the stem (1), said branches (2, 3, 4) being inscribed in the perimeter of the ellipsoid making up the section of the stem (1).

3. Surgical suction drain according to the preceding claim, **characterized in that** the branches (2, 3, 4) resulting from the division have a section reconstituting an ellipse with a large axis parallel to the small axis of the ellipse of the stem (1).

4. Surgical suction drain according to the preceding claim, **characterized in that** one of two adjacent branches (2, 3) has a convex wall and the other has a concave wall (7), provided to interlock with one another over at least part of their length.

5. Surgical suction drain according to any one of the preceding claims, **characterized in that** the branches (2, 3, 4) resulting from a separation have a section that becomes gradually circular.

6. Surgical suction drain according to any one of the preceding claims, **characterized in that** it is made from a flexible plastic material, such as silicone.

7. Surgical suction drain according to any one of the preceding claims, **characterized in that** the stem (1) comprises an end portion (10) with a circular section.
